(19) Europäisches Patentamt
European Patent Office
Office européen des brevets

(11) Veröffentlichungsnummer: **0 135 833**
**B1**

(12) # EUROPÄISCHE PATENTSCHRIFT

(45) Veröffentlichungstag der Patentschrift: **28.10.87**

(51) Int. Cl.⁴: **C 07 D 213/73**

(21) Anmeldenummer: **84110242.9**

(22) Anmeldetag: **29.08.84**

(54) **Verfahren zur Herstellung von 2-Amino-alkylpyridinen.**

(30) Priorität: **10.09.83 DE 3332687**

(43) Veröffentlichungstag der Anmeldung: **03.04.85 Patentblatt 85/14**

(45) Bekanntmachung des Hinweises auf die Patenterteilung: **28.10.87 Patentblatt 87/44**

(84) Benannte Vertragsstaaten: **BE CH DE FR GB IT LI**

(56) Entgegenhaltungen:
**EP - A - 0 062 542**
**EP - A - 0 082 613**
**US - A - 3 860 650**

(73) Patentinhaber: **BAYER AG, Konzernverwaltung RP Patentabteilung, D-5090 Leverkusen 1 Bayerwerk (DE)**

(72) Erfinder: **Le Blanc, Helmut, Dr., Neuenhaus 26, D-5093 Burscheid (DE)**
Erfinder: **Puppe, Lothar, Dr., Am Weiher 10a, D-5093 Burscheid (DE)**
Erfinder: **Wedemeyer, Karlfried, Dr., Bilharzstrasse 7, D-5000 Koeln 80 (DE)**

**EP 0 135 833 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

2-Amino-6-alkylpyridine können nach der Methode von Tschitschibabin durch Umsetzung von α-substituierten Pyridinen mit Natriumamid in inerten Lösungsmitteln hergestellt werden. Bei der Durchführung dieses Verfahrens ist wegen der Verwendung von Natriumamid auf völligen Wasserausschluss zu achten. Dies erfordert jedoch einen beträchtlichen sicherheitstechnischen Aufwand. Weiterhin ist bei der Reaktion nachteilig, dass das entstehende Natriumhydrid ebenfalls unter inerten Bedingungen vernichtet werden muss, was mit zusätzlichen Kosten verbunden ist.

Gemäss der japanischen Patentanmeldung Nr. 54/95 576 (1979), erhält man 2-Amino-6-methylpyridin durch Umsetzung von α-Picolin mit Ammoniak in Gegenwart von kobalthaltigen Katalysatoren. Bei dieser Umsetzung wird das 2-Amino-6-methylpyridin allerdings nur in sehr mässigen Ausbeuten erhalten.

Aus der US-PS 2 892 841 ist bekannt, 2-Amino-6-methylpyridin aus α-Picolin und Chloramin herzustellen. Nachteilig bei dieser Reaktion ist der Einsatz von Chloramin, das zur Zersetzung neigt und deshalb schwer zu handhaben ist, sowie die Isolierung des α-Amino-α′-picolins in einem mehrstufigen, umständlichen Verfahren.

Die Herstellung von α-Amino-pyridinen durch Umsetzung der entsprechenden Hydroxyverbindungen mit Ammoniak ist weiterhin aus der DE-OS 2 032 403 bekannt. Der Aminierungsschritt läuft im Falle des 2-Amino-6-methylpyridins mit nur 27% Ausbeute ab; ausserdem sind α-Alkyl-α′-hydroxypyridine, die als Ausgangsprodukte eingesetzt werden, nicht ohne weiteres zugänglich.

Aus US-PS 4 388 461 ist es bekannt, Ammoniak und Phenol an ZSM-5-Zeolithen bei 510 °C umzusetzen, wobei neben Anilin untergeordnete Mengen an α-Picolin erhalten werden. Daneben tritt eine grössere Zahl von Nebenprodukten, wie Toluidine, Xylidine und Diphenylamin auf, deren Entstehen offenbar auf die sehr hohe Reaktionstemperatur zurückzuführen ist. Diese Reaktionstemperatur ist mit hohem technischen Aufwand verbunden und lässt Verkokungen und häufigen Wechsel oder Regenerierung des Katalysators wahrscheinlich sein.

Ferner ist aus EP-PS 0 082 613 bekannt, Anilin an ZSM-5-Zeolithen bei 510 °C zu α-Picolin umzulagern. Selbst bei Anilinumsätzen von nur 8,3 bis 27,7% werden für α-Picolin nur Selektivitäten von 32,6–56,6% erreicht. Daneben entsteht eine Fülle anderer stickstoffhaltiger Verbindungen wie Toluidine, Xylidine, Diphenylamin, Indole und Chinoline. Neben dem schlechten Umsatz und der geringen Ausbeute an der gewünschten Substanz entstehen hier auch grosse Trennprobleme.

Es wurde nun ein Verfahren zur Herstellung von 2-Aminoalkylpyridinen der allgemeinen Formel

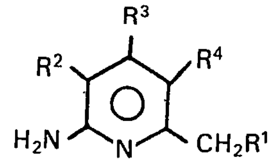

I,

worin

$R^1$ bis $R^4$ gleich oder verschieden sind und für Wasserstoff, $C_1$–$C_6$-Alkyl, $C_3$–$C_{12}$-Cycloalkyl, $C_7$–$C_{12}$-Aralkyl oder $C_6$–$C_{12}$-Aryl stehen, gefunden, das dadurch gekennzeichnet ist, dass man 1,3-Diaminobenzole der allgemeinen Formel

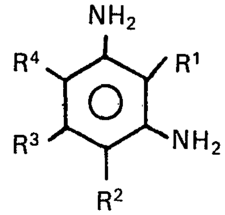

II,

worin

$R^1$ bis $R^4$ die oben genannte Bedeutung haben, in Gegenwart saurer Katalysatoren bei Temperaturen von 250 bis 500 °C.

Als Alkylreste seien bevorzugt solche mit 1 bis 4 Kohlenstoffatomen, genannt, wie der Methyl-, Ethyl-, n-Propyl-, iso-Propyl-, n-Butyl-, iso-Butyl-, tert.-Butyl-, n-Pentyl-, iso-Pentyl-, tert.-Pentyl- und der Hexylrest, bevorzugt der Methyl-, Ethyl-, n-Propyl- sowie der iso-Propylrest.

Als Cycloalkylreste seien bevorzugt solche mit 6 bis 8 Kohlenstoffatomen, wie der Cyclohexyl- und der Methylcyclohexylrest, bevorzugt der Cyclohexylrest genannt; als Aralkylreste bevorzugt solche mit 7 bis 9 Kohlenstoffatomen, wie der Benzyl- und der Methylbenzylrest, bevorzugt der Benzylrest, und als Arylreste bevorzugt solche mit 6 bis 9 Kohlenstoffatomen, wie der Phenyl-, der Tolyl- und der Trimethylphenylrest, bevorzugt der Phenyl- und der Tolylrest.

Zum Beispiel können in das erfindungsgemässe Verfahren 1,3-Diaminobenzol, 2,6-Diaminotoluol, 2,4-Diaminotoluol, 3,5-Diaminocumol bevorzugt 1,3-Diaminobenzol, eingesetzt werden.

Das erfindungsgemässe Verfahren wird in einem Temperaturbereich von 250 bis 500 °C, bevorzugt bei 300 bis 450 °C und besonders bevorzugt bei 350 bis 400 °C durchgeführt. Das erfindungsgemässe Verfahren kann sowohl bei Normaldruck als auch bei erhöhtem Druck, beispielsweise bei 10 bis 250 bar, bevorzugt 30 bis 190 bar, durchgeführt werden.

In das erfindungsgemässe Verfahren werden saure Katalysatoren eingesetzt. Es eignen sich sowohl Protonensäuren als auch Lewissäuren. Zum Beispiel werden genannt: Homo- und Heteropolysäuren, Zeolithe, Alumosilikate sowie Aluminiumoxide. In vorteilhafter Weise kann die erfindungsgemässe Isomerisierung mit Zeolithen, Alumosilikaten und/oder γ-Aluminiumoxid durchgeführt werden. Besonders gut geeignet sind Zeolithe mit einer ZSM-5-Struktur, wie sie in der US-PS 3 702 886 beschrieben sind.

Zur Durchführung des erfindungsgemässen Verfahrens wird das entsprechende 1,3-Diaminobenzol üblicherweise in einem inerten Lösungs- und/oder Verdünnungsmittel, wie Wasser, Toluol und/oder Ammoniak, bevorzugt in Ammoniak aufgenommen, wobei das Lösungs- und/oder Verdünnungsmittel im allgemeinen im Überschuss eingesetzt wird. Die jeweils einzusetzende Menge an Lösungs- und/oder Verdünnungsmittel ist nicht kritisch und kann in weiten Bereichen schwanken. Die geeignete Menge kann leicht durch Vorversuche ermittelt werden.

Das erfindungsgemässe Verfahren kann sowohl in der flüssigen Phase als auch in der Gasphase durchgeführt werden, bevorzugt wird in der Gasphase gearbeitet. Dabei kann zur Erhöhung der Strömungsgeschwindigkeit dem Reaktionsgemisch ein inertes Gas, wie Stickstoff und/oder Argon, zudosiert werden.

Die erfindungsgemässe Umsetzung kann sowohl diskontinuierlich als auch kontinuierlich durchgeführt werden.

Zur Durchführung des erfindungsgemässen Verfahrens wird beispielsweise ein Gemisch aus Ammoniak und dem entsprechenden 1,3-Diaminobenzol unter Druck über den Katalysator geleitet. Nach dem Verlassen des Reaktors wird das Reaktionsgemisch in an sich bekannter Weise, z.B. durch fraktionierte Destillation, aufgearbeitet. Das noch nicht umgesetzte 1,3-Diaminobenzol kann praktisch ohne Verluste wieder in die Reaktion zurückgeführt werden.

Das erfindungsgemässe Verfahren lässt sich durch das folgende allgemeine Formelschema darstellen (am Beispiel des 1,3-Diaminobenzols):

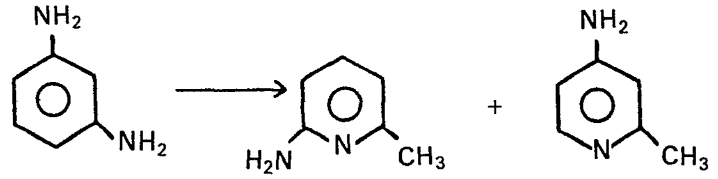

Wie dem Formelschema zu entnehmen ist, entsteht bei der erfindungsgemässen Reaktion neben dem 2-Amino-alkylpyridin stets eine gewisse Menge an 4-Amino-alkylpyridin. In welcher Menge dabei das 4-Amino-alkylpyridin gebildet wird, ist u.a. abhängig von den jeweils angewendeten Reaktionsbedingungen sowie von dem eingesetzten Diaminobenzol.

Nach dem erfindungsgemässen Verfahren ist es auch möglich statt der m-Phenylendiamine Resorcin oder entsprechend substituierte Resorcine oder m-Aminophenol oder entsprechend substituierte m-Aminophenole einzusetzen. In diesem Fall ist es erforderlich, dass die Reaktion in einem ammoniakalischen Medium durchgeführt wird. Dabei wird Ammoniak in einem Überschuss von 2 bis 150 Mol, bevorzugt 5 bis 120 Mol, besonders bevorzugt 10 bis 60 Mol, pro Mol eingesetztes Resorcin oder Aminophenol eingesetzt. Statt reinem Ammoniak kann auch eine wässrig-ammoniakalische Lösung verwendet werden, z.B. eine 30%ige wässrig-ammoniakalische Lösung.

Die Vorteile des erfindungsgemässen Verfahrens sind sowohl in der hohen Selektivität zu sehen, mit der die 2-Amino-alkylpyridine gebildet werden, als auch in den guten Ausbeuten an 2-Amino-alkylpyridinen.

Das erfindungsgemässe Verfahren eignet sich insbesondere zur technischen Herstellung von 2-Amino-6-alkylpyridinen, weil die eingesetzten m-Phenylendiamine und besonders das 1,3-Diaminobenzol selbst gut zugängliche und leicht zu handhabende organische Grundchemikalien sind und das nicht umgesetzte Einsatzprodukt leicht vom Endprodukt abdestilliert und praktisch verlustfrei in die Reaktion zurückgeführt werden kann. Darüber hinaus ist es von Vorteil, dass das erfindungsgemässe Verfahren ohne besondere Sicherheitsmassnahmen durchzuführen ist und, da kein Abwasser anfällt, sich besonders umweltfreundlich darstellt.

Diese Vorteile sind deshalb überraschend, als nach dem Stande der Technik, besonders unter Berücksichtigung von US-PS 4 388 461 und EP-PS 82 613, eine Anzahl von Nebenprodukten zu erwarten gewesen wäre, dies umso mehr als die erfindungsgemäss einzusetzenden, gegebenenfalls substituierten 1,3-Diaminobenzole 2 statt einer Aminogruppe tragen. Solche Nebenprodukte hätten zum Beispiel gegebenenfalls substituierte Heterocyclen mit 2 N-Atomen im Kern sein können. Überraschend ist ferner die im Vergleich zur Umlagerung von Anilin, das auch aus Phenol und $NH_3$ entstanden sein kann, wesentlich tiefere Reaktionstemperatur. Überraschend ist schliesslich die hohe Spezifität der Reaktion, denn die entsprechenden o- und p-Diaminobenzole reagieren nicht in der beschriebenen Weise.

2-Amino-alkylpyridine finden Verwendung als Ausgangsprodukte für Pflanzenschutzmittel und für Pharmazeutika. Beispielsweise stellt das 2-Amino-6-methylpyridin ein wichtiges Zwischenprodukt zur Herstellung von pharmazeutischen Wirkstoffen dar, wie der Nalidixinsäure (vgl. J. Med. Pharm. Che. 5, 1063 (1962) ).

Das erfindungsgemässe Verfahren soll anhand der folgenden Beispiele näher erläutert werden.

Beispiel 1

Ein Reaktor von 76 cm Länge und 1 cm Durchmesser, der mit 60 ml Katalysator gefüllt war, wurde in einem Salzbad auf 350 °C aufgeheizt. Bei dem Katalysator handelte es sich um einen Zeolithen mit einer ZSM-5-Struktur (vgl. US-PS

3 702 886). Der Katalysator besass eine Korngrösse von 0,5 bis 1,0 mm.

In einem Vorverdampfer verdampfte man ein Gemisch aus 12,1 g Ammoniak und 1,3 g 1,3-Diaminobenzol in der Stunde (Molverhältnis 60:1) und leitete das auf Reaktionstemperatur gebrachte Gasgemisch bei einem Druck von 190 bar durch den Reaktor. Das entspannte Gemisch wurde anschliessend kondensiert und gaschromatographisch analysiert. Das Produkt enthielt:

35,8% 2-Amino-6-methylpyridin
6,6% 2-Methyl-4-aminopyridin
56,8% 1,3-Diaminobenzol

Die Konfiguration der Aminopyridine wurde NRM-spektroskopisch bestätigt.

Schmelzpunkte Fp: 2-Amino-6-methylpyridin 44,5–45° (Lit.: 41°)

Fp: 2-Methyl-4-aminopyridin 95° (Lit.: 95–96°)

Beispiel 2

Die Arbeitsweise von Beispiel 1 wurde mit der Änderung wiederholt, dass ein Gemisch aus 11,4 g Ammoniak und 1,2 g 1,3-Diaminobenzol (Molverhältnis 60:1) pro Stunde durch den Reaktor bei einer Temperatur von 400 °C geleitet wurde. Das Produktgemisch enthielt:

56,4% 2-Amino-6-methylpyridin
15,4% 2-Methyl-4-aminopyridin
26,6% 1,3-Diaminobenzol

Beispiel 3

Die Arbeitsweise von Beispiel 1 wurde mit der Änderung wiederholt, dass ein Gemisch aus 7,4 g Ammoniak und 4,7 g 1,3-Diaminobenzol pro Stunde (Molverhältnis 10:1) durch den Reaktor geleitet wurde. Das Produkt enthielt:

11,0% 2-Amino-6-methylpyridin
2,6% 2-Methyl-4-aminopyridin
86,2% 1,3-Diaminobenzol

Beispiel 4

Die Arbeitsweise von Beispiel 1 wurde mit der Änderung wiederholt, dass ein Gemisch aus 16,3 g Ammoniak und 1,7 g 1,3-Diaminobenzol pro Stunde (Molverhältnis 60:1) bei einem Druck von 30 bar durch den Reaktor geleitet wurde. Das Produktgemisch enthielt:

13,1% 2-Amino-6-methylpyridin
3,8% 2-Methyl-4-aminopyridin
81,4% 1,3-Diaminobenzol

Beispiel 5

Die Arbeitsweise von Beispiel 1 wurde mit der Änderung wiederholt, dass ein Gemisch aus 12,1 g Ammoniak und 1,3 g 1,3-Diaminobenzol pro Stunde (Molverhältnis 60:1) durch den Reaktor, der mit einem Aluminiumsilikat (LA-5P der Firma Akzo), das ca. 15% $Al_2O_3$ und 85% $SiO_2$ enthält, gefüllt war, geleitet wurde. Das Produktgemisch enthielt:

8,9% 2-Amino-6-methylpyridin
1,3% 2-Methyl-4-aminopyridin
84,2% 1,3-Diaminobenzol

Beispiel 6

Die Arbeitsweise von Beispiel 1 wurde mit der Änderung wiederholt, dass ein Gemisch aus 10,1 g Ammoniak und 1,1 g 3-Aminophenol (Molverhältnis 60:1) pro Stunde durch den Reaktor, der mit hochreinem γ-Aluminiumoxid (D 10–10 der Firma BASF) gefüllt war, bei einer Temperatur von 400 °C geleitet wurde. Das Produktgemisch enthielt:

29,1% 2-Amino-6-methylpyridin
68,4% 1,3-Diaminobenzol

Beispiel 7

Die Arbeitsweise von Beispiel 1 wurde mit der Änderung wiederholt, dass ein Gemisch aus 11,4 g Ammoniak und 1,2 g 3-Aminophenol (Molverhältnis 60:1) pro Stunde bei einem Druck von 30 bar durch den Reaktor, der mit γ-Aluminiumoxid (D 10–10) gefüllt war, bei einer Temperatur von 350 °C geleitet wurde. Das Produktgemisch enthielt:

25,4% 2-Amino-6-methylpyridin
71,6% 1,3-Diaminobenzol

Beispiel 8

Die Arbeitsweise von Beispiel 1 wurde mit der Änderung wiederholt, dass ein Gemisch aus 11,4 g Ammoniak und 1,2 g Resorcin (Molverhältnis 60:1) pro Stunde durch den Reaktor, der mit γ-Aluminiumoxid (D 10–10) gefüllt war, bei einer Temperatur von 320 °C geleitet wurde. Das Produktgemisch enthielt:

15% 2-Amino-6-methylpyridin
84% 1,3-Diaminobenzol

Beispiel 9

Die Arbeitsweise von Beispiel 1 wurde mit der Änderung wiederholt, dass ein Gemisch aus 11,4 g Ammoniak, 1,3 g Wasser und 1,2 g 3-Aminophenol pro Stunde durch den Reaktor, der mit hochreinem γ-Aluminiumoxid (BR 1597 der Firma Kali Chemie) gefüllt war, bei einer Temperatur von 380 °C geleitet wurde. Das Produktgemisch enthielt:

24,0% 2-Amino-6-methylpyridin
71,0% 1,3-Diaminobenzol

Beispiel 10

Die Arbeitsweise von Beispiel 1 wurde mit der Änderung wiederholt, dass ein Gemisch aus 3,5 g Ammoniak und 0,4 g 2,6-Diaminotoluol (Molverhältnis 60:1) pro Stunde durch den Reaktor, der mit γ-Aluminiumoxid (D 10–10) gefüllt war, geleitet wurde. Das Produktgemisch enthielt:

14,9% 2-Amino-6-methylpyridin
83,5% 1,3-Diaminobenzol

Beispiel 11

Die Arbeitsweise von Beispiel 1 wurde mit der Änderung wiederholt, dass ein Gemisch aus 3,6 g Ammoniak und 0,4 g 2,4-Diaminotoluol (Molverhältnis 60:1) pro Stunde durch den Reaktor, der mit γ-Aluminiumoxid (D 10–10) gefüllt war,

geleitet wurde. Das Produktgemisch enthielt:
7,1% 2-Amino-5,6-dimethylpyridin
11,6% 2-Amino-3,6-dimethylpyridin
73,5% 2,4-Diaminotoluol

Beispiel 12

Die Arbeitsweise von Beispiel 1 wurde mit der Änderung wiederholt, dass ein Gemisch aus 15,2 g Ammoniak und 1,5 g 3,5-Diaminocumol (Molverhältnis 90:1) pro Stunde durch den Reaktor, der mit $\gamma$-Aluminiumoxid (BR 1597) gefüllt war, geleitet wurde. Das Produktgemisch enthielt:
10,1% 2-Amino-4-isopropyl-6-methylpyridin
86,7% 3,5-Diaminocumol
Fp. 2-Amino- 4-isopropyl- 6-methylpyridin 59 °C
Fp. 3,5-Diaminocumol 61 °C

Beispiel 13

In in einem 0,7 Liter Stahlautoklaven wurde ein Gemisch aus 60 g 1,3-Diaminobenzol, 80 g Ammoniak und 30 g Zeolith (ZSM-5-Pulver) 12 Stunden bei 350 °C gerührt.

Das im Hochvakuum destillierte Rohprodukt (Ausbeute Destillat: 47,8 g = 80% der Theorie) enthielt:
8,2% 2-Amino-6-methylpyridin
2,3% 2-Methyl-4-aminopyridin
88,8% 1,3-Diaminobenzol

Beispiel 14

Die Arbeitsweise von Beispiel 1 wurde mit der Änderung wiederholt, dass ein Gemisch aus 11,5 g $NH_3$ und 1,2 g 1,3-Diaminobenzol pro Stunde bei einer Temperatur von 380 °C durch den Reaktor geleitet wurde. Das erhaltene Produktgemisch enthielt:
41,1% 2-Amino-6-methylpyridin
13,6% 2-Methyl-4-aminopyridin
44,1% 1,3-Diaminobenzol

Beispiel 15 (zum Vergleich)

Die Arbeitsweise von Beispiel 1 wurde mit der Änderung wiederholt, dass ein Gemisch aus 9,9 g $NH_3$ und 0,9 g Anilin pro Stunde bei einer Temperatur von 380 °C durch den Reaktor geleitet wurde. Das erhaltene Produktgemisch enthielt:
2,9% $\alpha$-Picolin
96,7% Anilin
Bei 350 °C erhielt man unter sonst gleichbleibenden Bedingungen weniger als 1% $\alpha$-Picolin.

## Patentansprüche

1. Verfahren zur Herstellung von 2-Amino-alkylpyridinen der Formel

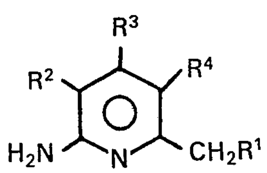

worin

$R^1$ bis $R^4$ gleich oder verschieden sind und für Wasserstoff, $C_1$–$C_6$-Alkyl, $C_3$–$C_{12}$-Cycloalkyl, $C_7$–$C_{12}$-Aralkyl oder $C_6$–$C_{12}$-Aryl stehen, dadurch gekennzeichnet, dass man 1,3-Diaminobenzole der Formel

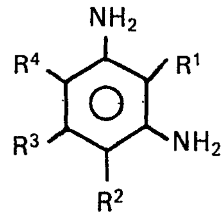

worin

$R^1$ bis $R^4$ die obengenannte Bedeutung haben, in Gegenwart saurer Katalysatoren bei Temperaturen von 250 bis 500 °C isomerisiert.

2. Verfahren nach Anspruch 1, dadurch gekennzeichnet, dass die Isomerisierung in Gegenwart von Protonen- und/oder Lewissäuren durchgeführt wird.

3. Verfahren nach Ansprüchen 1 und 2, dadurch gekennzeichnet, dass die Isomerisierung in Gegenwart von Zeolithen, Alumosilikaten und/oder $\gamma$-Aluminiumoxid durchgeführt wird.

4. Verfahren nach Ansprüchen 1 bis 3, dadurch gekennzeichnet, dass man die Isomerisierung in Gegenwart von Zeolithen mit einer ZSM-5-Struktur durchführt.

5. Verfahren nach Ansprüchen 1 bis 4, dadurch gekennzeichnet, dass man die Isomerisierung bei Temperaturen von 350 bis 400 °C durchführt.

6. Verfahren nach Ansprüchen 1 bis 5, dadurch gekennzeichnet, dass man statt der 1,3-Diaminobenzole die entsprechenden 1,3-Dihydroxybenzole oder die entsprechenden m-Aminophenole einsetzt und die Isomerisierung in einem ammoniakalischen Medium durchführt.

## Revendications

1. Procédé de production de 2-aminoalkylpyridines de formule

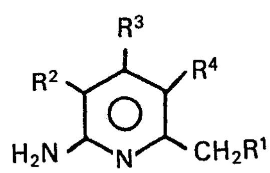

dans laquelle

$R^1$ à $R^4$ sont identiques ou différents et représentent de l'hydrogène, des groupes alkyle en $C_1$ à $C_6$, cycloalkyle en $C_3$ à $C_{12}$, aralkyle en $C_7$ à $C_{12}$ ou aryle en $C_6$ à $C_{12}$, caractérisé en ce qu'on isomérise à des températures de 250 à 500 °C, en présence de catalyseurs acides, des 1,3-diaminobenzènes de formule

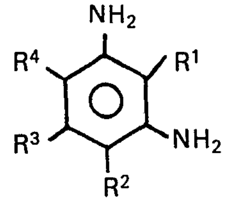

dans laquelle

$R^1$ à $R^4$ ont la définition indiquée ci-dessus.

2. Procédé suivant la revendication 1, caractérisé en ce que l'isomérisation est conduite en présence d'acides protoniques et/ou d'acides de Lewis.

3. Procédé selon les revendications 1 et 2, caractérisé en ce qu'on conduit l'isomérisation en présence de zéolites, d'aluminosilicates et/ou d'oxyde d'aluminium-γ.

4. Procédé suivant les revendications 1 à 3, caractérisé en ce qu'on conduit l'isomérisation en présence de zéolites à structure de ZSM-5.

5. Procédé suivant les revendications 1 à 4, caractérisé en ce qu'on conduit l'isomérisation à des températures de 350 à 400 °C.

6. Procédé suivant les revendications 1 à 5, caractérisé en ce qu'on utilise à la place des 1,3-diaminobenzènes, les 1,3-dihydroxybenzènes correspondants ou les m-aminophénols correspondants, et en ce qu'on conduit l'isomérisation dans un milieu ammoniacal.

## Claims

1. Process for the preparation of 2-amino-alkylpyridines of the formula

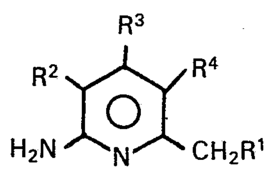

wherein

$R^1$ to $R^4$ are identical or different and represent hydrogen, $C_1$–$C_6$-alkyl, $C_3$–$C_{12}$-cycloalkyl, $C_7$–$C_{12}$-aralkyl or $C_6$–$C_{12}$-aryl, characterized in that 1,3-diaminobenzenes of the formula

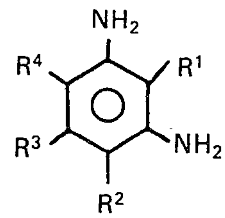

wherein

$R^1$ to $R^4$ have the above-mentioned meaning, are isomerised in the presence of acid catalysts at temperatures of 250 to 500 °C.

2. Process according to Claim 1, characterized in that the isomerisation is carried out in the presence of protonic acids and/or Lewis acids.

3. Process according to Claims 1 and 2, characterized in that the isomerisation is carried out in the presence of zeolites, aluminosilicates and/or γ-aluminium oxide.

4. Process according to Claims 1 to 3, characterized in that the isomerisation is carried out in the presence of zeolites with a ZSM-5-structure.

5. Process according to Claims 1 to 4, characterized in that the isomerisation is carried out at temperatures of 350 to 400 °C.

6. Process according to Claims 1 to 5, characterized in that, instead of the 1,3-diaminobenzenes, the corresponding 1,3-dihydroxybenzenes or the corresponding m-aminophenols are used and the isomerisation is carried out in an ammoniacal medium.